# EUROPEAN PATENT APPLICATION

(11) **EP 2 308 999 A1**
(43) Date of publication of application: **13.04.2011**
(21) Application number: 09012160.9
(22) Date of filing: 24.09.2009
(51) Int. Cl.: C12Q 1/68, G01N 33/50

(54) **A combination of markers for predicting the mortality risk in a polytraumatized patient**

(71) Applicant: Ludwig-Maximilians-Universität München, 80359 München (DE)
(72) Inventor: Biberthaler, Peter, 82131 Gauting (DE); Bogner, Viktoria, 85579 Neubiberg (DE); Giese, Thomas, 69198 Schriesheim (DE)
(74) Representative: Engelhard, Markus

(57) **Abstract**

The present invention relates to a combination of markers selected from MMP-9, BCL2A1 and ETS-2 for use in predicting the mortality risk in a patient suffering from polytrauma. The present invention also relates to a kit for predicting the mortality risk in such patient, and to a method of predicting the mortality risk in such patient. Furthermore, the present invention relates to a nucleic acid composition, to a matrix on which such nucleic acid composition has been immobilized. Furthermore, the present invention relates to uses of the aforementioned kit, of the nucleic acid composition and of the matrix.

SEQUENCE LISTING

<110> Ludwig-Maximilians-Universität München

<120> A combination of markers for predicting the moratlity risk in a
polytraumatized patient

<130> FB21524

<160> 5

<170> PatentIn version 3.5

<210> 1
<211> 899
<212> DNA
<213> Homo sapiens

<400> 1
agcctacgca cgaaagtgac taggaggaag gatattataa agtgatgcaa acagaaattc 60

caccagcctc catgtatcat catgtgtcat aactcagtca agctcagtga gcattctcag 120

cacattgcct caacagcttc aaggtgagcc agctcaagac tttgctctcc accaggcaga 180

agatgacaga ctgtgaattt ggatatattt acaggctggc tcaggactat ctgcagtgcg 240

tcctacagat accacaacct ggatcaggtc caagcaaaac gtccagagtg ctacaaaatg 300

ttgcgttctc agtccaaaaa gaagtggaaa agaatctgaa gtcatgcttg gacaatgtta 360

atgttgtgtc cgtagacact gccagaacac tattcaacca agtgatggaa aaggagtttg 420

aagacggcat cattaactgg ggaagaattg taaccatatt tgcatttgaa ggtattctca 480

tcaagaaact tctacgacag caaattgccc cggatgtgga tacctataag gagatttcat 540

attttgttgc ggagttcata atgaataaca caggagaatg gataaggcaa aacggaggct 600

gggaaaatgg ctttgtaaag aagtttgaac ctaaatctgg ctggatgact tttctagaag 660

ttacaggaaa gatctgtgaa atgctatctc tcctgaagca atactgttga ccagaaagga 720

cactccatat tgtgaaaccg gcctaatttt tctgactgat atggaaacga ttgccaacac 780

atacttctac ttttaaataa acaactttga tgatgtaact tgaccttcca gagttatgga 840

aattttgtcc ccatgtaatg aataaattgt atgtattttt ctctataaaa aaaaaaaaa 899

<210> 2
<211> 2387
<212> DNA
<213> Homo sapiens

<400> 2
agacacctct gccctcacca tgagcctctg gcagcccctg gtcctggtgc tcctggtgct 60

gggctgctgc tttgctgccc ccagacagcg ccagtccacc cttgtgctct tccctggaga 120

cctgagaacc aatctcaccg acaggcagct ggcagaggaa tacctgtacc gctatggtta 180

cactcgggtg gcagagatgc gtggagagtc gaaatctctg gggcctgcgc tgctgcttct 240

ccagaagcaa ctgtccctgc ccgagaccgg tgagctggat agcgccacgc tgaaggccat 300

gcgaacccca cggtgcgggg tcccagacct gggcagattc caaacctttg agggcgacct 360

caagtggcac caccacaaca tcacctattg gatccaaaac tactcggaag acttgccgcg 420

ggcggtgatt gacgacgcct ttgcccgcgc cttcgcactg tggagcgcgg tgacgccgct 480

caccttcact cgcgtgtaca gccgggacgc agacatcgtc atccagtttg gtgtcgcgga 540

gcacggagac gggtatccct tcgacgggaa ggacgggctc ctggcacacg cctttcctcc 600

tggccccggc attcagggag acgcccattt cgacgatgac gagttgtggt ccctgggcaa 660

gggcgtcgtg gttccaactc ggtttggaaa cgcagatggc gcggcctgcc acttcccctt 720

catcttcgag ggccgctcct actctgcctg caccaccgac ggtcgctccg acggcttgcc 780

ctggtgcagt accacggcca actacgacac cgacgaccgg tttggcttct gccccagcga 840

gagactctac acccaggacg gcaatgctga tgggaaaccc tgccagtttc cattcatctt 900

ccaaggccaa tcctactccg cctgcaccac ggacggtcgc tccgacggct accgctggtg 960

cgccaccacc gccaactacg accgggacaa gctcttcggc ttctgcccga cccgagctga 1020

ctcgacggtg atgggggcca actcggcggg ggagctgtgc gtcttcccct tcactttcct 1080

gggtaaggag tactcgacct gtaccagcga gggccgcgga gatgggcgcc tctggtgcgc 1140

taccacctcg aactttgaca gcgacaagaa gtggggcttc tgcccggacc aaggatacag 1200

tttgttcctc gtggcggcgc atgagttcgg ccacgcgctg ggcttagatc attcctcagt 1260

gccggaggcg ctcatgtacc ctatgtaccg cttcactgag gggcccccct tgcataagga 1320

cgacgtgaat ggcatccggc acctctatgg tcctcgccct gaacctgagc cacggcctcc 1380

aaccaccacc acaccgcagc ccacggctcc cccgacggtc tgccccaccg gaccccccac 1440

tgtccacccc tcagagcgcc ccacagctgg ccccacaggt cccccctcag ctggccccac 1500

aggtcccccc actgctggcc cttctacggc cactactgtg cctttgagtc cggtggacga 1560

tgcctgcaac gtgaacatct tcgacgccat cgcggagatt gggaaccagc tgtatttgtt 1620

caaggatggg aagtactggc gattctctga gggcaggggg agccggccgc agggcccctt 1680

ccttatcgcc gacaagtggc ccgcgctgcc ccgcaagctg gactcggtct ttgaggagcg 1740

gctctccaag aagcttttct tcttctctgg gcgccaggtg tgggtgtaca caggcgcgtc 1800

ggtgctgggc ccgaggcgtc tggacaagct gggcctggga gccgacgtgg cccaggtgac 1860

cggggccctc cggagtggca gggggaagat gctgctgttc agcgggcggc gcctctggag 1920

gttcgacgtg aaggcgcaga tggtggatcc ccggagcgcc agcgaggtgg accggatgtt 1980

ccccggggtg cctttggaca cgcacgacgt cttccagtac cgagagaaag cctatttctg 2040

ccaggaccgc ttctactggc gcgtgagttc ccggagtgag ttgaaccagg tggaccaagt 2100

gggctacgtg acctatgaca tcctgcagtg ccctgaggac tagggctccc gtcctgcttt 2160

ggcagtgcca tgtaaatccc cactgggacc aaccctgggg aaggagccag tttgccggat 2220

acaaactggt attctgttct ggaggaaagg gaggagtgga ggtgggctgg gccctctctt 2280

ctcacctttg ttttttgttg gagtgtttct aataaacttg gattctctaa cctttaaaaa 2340

aaaaaaaaaa aaaaaaaaaa aaaaaaaaaa aaaaaaaaaa aaaaaaa 2387

<210> 3
<211> 3672
<212> DNA
<213> Homo sapiens

<400> 3
gcccggttac ttcctccaga gactgacgag tgcggtgtcg ctccagctca gagctcccgg 60

agccgcccgg ccagcgtccg gcctccctga tcgtctctgg ccggcgccct cgccctcgcc 120

cggcgcgcac cgagcagccg cgggcgccga gcagccaccg tcccgaccaa gcgccggccc 180

tgcccgcagc ggcaggatga atgatttcgg aatcaagaat atggaccagg tagcccctgt 240

ggctaacagt tacagaggga cactcaagcg ccagccagcc tttgacacct ttgatgggtc 300

cctgtttgct gtttttcctt ctctaaatga agagcaaaca ctgcaagaag tgccaacagg 360

cttggattcc atttctcatg actccgccaa ctgtgaattg cctttgttaa ccccgtgcag 420

caaggctgtg atgagtcaag ccttaaaagc taccttcagt ggcttcaaaa aggaacagcg 480

gcgcctgggc attccaaaga acccctggct gtggagtgag caacaggtat gccagtggct 540

tctctgggcc accaatgagt tcagtctggt gaacgtgaat ctgcagaggt tcggcatgaa 600

tggccagatg ctgtgtaacc ttggcaagga acgctttctg gagctggcac ctgactttgt 660

gggtgacatt ctctgggaac atctggagca aatgatcaaa gaaaaccaag aaaagacaga 720

agatcaatat gaagaaaatt cacacctcac ctccgttcct cattggatta acagcaatac 780

attaggtttt ggcacagagc aggcgcccta tggaatgcag acacagaatt accccaaagg 840

cggcctcctg gacagcatgt gtccggcctc cacacccagc gtactcagct ctgagcagga 900

gtttcagatg ttccccaagt ctcggctcag ctccgtcagc gtcacctact gctctgtcag 960

tcaggacttc ccaggcagca acttgaattt gctcaccaac aattctggga ctcccaaaga 1020

ccacgactcc cctgagaacg gtgcggacag cttcgagagc tcagactccc tcctccagtc 1080

ctggaacagc cagtcgtcct tgctggatgt gcaacgggtt ccttccttcg agagcttcga 1140

agatgactgc agccagtctc tctgcctcaa taagccaacc atgtctttca aggattacat 1200

ccaagagagg agtgacccag tggagcaagg caaaccagtt atacctgcag ctgtgctggc 1260

cggcttcaca ggaagtggac ctattcagct gtggcagttt ctcctggagc tgctatcaga 1320

caaatcctgc cagtcattca tcagctggac tggagacgga tgggagttta agctcgccga 1380

ccccgatgag gtggcccgcc ggtggggaaa gaggaaaaat aagcccaaga tgaactacga 1440

gaagctgagc cggggcttac gctactatta cgacaagaac atcatccaca agacgtcggg 1500

gaagcgctac gtgtaccgct tcgtgtgcga cctccagaac ttgctggggt tcacgcccga 1560

ggaactgcac gccatcctgg gcgtccagcc cgacacggag gactgaggtc gccgggacca 1620

ccctgagccg gccccaggct cgtggactga gtgggaagcc catcctgacc agctgctccg 1680

aggacccagg aaaggcagga ttgaaaatgt ccaggaaagt ggccaagaag cagtggcctt 1740

attgcatccc aaaccacgcc tcttgaccag gctgcctccc ttgtggcagc aacggcacag 1800

ctaattctac tcacagtgct tttaagtgaa aatggtcgag aaagaggcac caggaagccg 1860

tcctggcgcc tggcagtccg tgggacggga tggttctggc tgtttgagat tctcaaagga 1920

gcgagcatgt cgtggacaca cacagactat ttttagattt tcttttgcct tttgcaacca 1980

ggaacagcaa atgcaaaaac tctttgagag ggtaggaggg tgggaaggaa acaaccatgt 2040

catttcagaa gttagtttgt atatattatt ataatcttat aattgttctc agaatccctt 2100

aacagttgta tttaacagaa attgtatatt gtaatttaaa ataattatat aactgtattt 2160

gaaataagaa ttcagacatc tgaggtttta tttcattttt caatagcaca tatggaattt 2220

tgcaaagatt taatctgcca agggccgact aagagaagtt gtaaagtatg tattatttac 2280

atttaataga cttacaggga taaggcctgt ggggggtaat ccctgctttt tgtgtttttt 2340

tgtttgtttg tttgtttgtt tttggggggt tttcttgcct tggttgtctg gcaaggactt 2400

tgtacatttg ggagttttta tgagaaactt aaatgttatt atctgggctt atatctggcc 2460

tctgctttct cctttaattg taaagtaaaa gctataaagc agtatttttc ttgacaaatg 2520

gcatatgttt tccacttctt tgcatgcgtt taagtcagtt tatacacaaa atggatttta 2580

ttttttagtt taactgtgtt tctccgacag ctcacctctc tctgaccacc cagccatttc 2640

cttcctgtgc tccacgttct tctgtgtgat taaaataaga atattatttt tggaaatatg 2700

caactccttt tcagagatca ggagggattt atgtagcagc tatttttact gcaaaagtaa 2760

ttcactggaa aaaaaatgta atttgtaaga aagctttatt tttatctcag ctctatgtaa 2820

agttaaagtt actgtacaga gctgaaggac ggggggcggt aggggtcttg atgaaacctc 2880

ttgaacgaag cacagtttgt cccatctttg ttcactcgtg tgtctcaacc atcttaatag 2940

catgctgctc ctttttgctc agtgtccaca gcaagatgac gtgattctta ttttcttgga 3000

cacagactat tctgaggcac agagcgggga cttaagatgg gaaagagaaa gcatcggagc 3060

cattcattcg gagaaaacgt tttgatcaaa atggagactt ttgtagtcgt ttcaaaagag 3120

cacctgagtc atgtgtattc ccggccttta taaatgaccc ggtcaagttg gtttcaaagt 3180

ccgacaggct tgtctgttta ctagctgcgt ggccttggac gggtggctga catctgtaaa 3240

gaatcctcct gtgatgaaac tgaggaatcg ggtggccggg caagctggga agagcaaagc 3300

cagagctgcg ctgcctcaat acccacaaaa gaccattccc agtatacata agcacaggat 3360

gtttttctca agagggatgt atttatcact tggacatctg tttataatat aaacagacat 3420

gtgactggga acatcttgct gccaaaagaa tcctaggcag tggctcattg tatgtgaggt 3480

tgaaccacgt gaaattgcca atattaggct ggcttttatc tacaaagaag gagtttcatg 3540

gggttcagcc taacagttat ggaaactaca gtccttataa accattggca tggtaataaa 3600

cagatcttaa gtataaaaat tttgtaattg ggcctttact ctctcaataa taaagtattt 3660

tgtttatata aa 3672

<210> 4
<211> 1852
<212> DNA
<213> Homo sapiens

<400> 4
accgccgaga ccgcgtccgc cccgcgagca cagagcctcg cctttgccga tccgccgccc 60

gtccacaccc gccgccagct caccatggat gatgatatcg ccgcgctcgt cgtcgacaac 120

ggctccggca tgtgcaaggc cggcttcgcg ggcgacgatg ccccccgggc cgtcttcccc 180

tccatcgtgg ggcgccccag gcaccagggc gtgatggtgg gcatgggtca gaaggattcc 240

tatgtgggcg acgaggccca gagcaagaga ggcatcctca ccctgaagta ccccatcgag 300

cacggcatcg tcaccaactg ggacgacatg gagaaaatct ggcaccacac cttctacaat 360

gagctgcgtg tggctcccga ggagcacccc gtgctgctga ccgaggcccc cctgaacccc 420

aaggccaacc gcgagaagat gacccagatc atgtttgaga ccttcaacac cccagccatg 480

tacgttgcta tccaggctgt gctatccctg tacgcctctg gccgtaccac tggcatcgtg 540

atggactccg gtgacggggt cacccacact gtgcccatct acgaggggta tgccctcccc 600

catgccatcc tgcgtctgga cctggctggc cgggacctga ctgactacct catgaagatc 660

ctcaccgagc gcggctacag cttcaccacc acggccgagc gggaaatcgt gcgtgacatt 720

aaggagaagc tgtgctacgt cgccctggac ttcgagcaag agatggccac ggctgcttcc 780

agctcctccc tggagaagag ctacgagctg cctgacggcc aggtcatcac cattggcaat 840

gagcggttcc gctgccctga ggcactcttc cagccttcct tcctgggcat ggagtcctgt 900

ggcatccacg aaactacctt caactccatc atgaagtgtg acgtggacat ccgcaaagac 960

ctgtacgcca acacagtgct gtctggcggc accaccatgt accctggcat tgccgacagg 1020

atgcagaagg agatcactgc cctggcaccc agcacaatga agatcaagat cattgctcct 1080

cctgagcgca agtactccgt gtggatcggc ggctccatcc tggcctcgct gtccaccttc 1140

cagcagatgt ggatcagcaa gcaggagtat gacgagtccg gcccctccat cgtccaccgc 1200

aaatgcttct aggcggacta tgacttagtt gcgttacacc ctttcttgac aaaacctaac 1260

ttgcgcagaa aacaagatga gattggcatg gctttatttg ttttttttgt tttgttttgg 1320

tttttttttt ttttttggct tgactcagga tttaaaaact ggaacggtga aggtgacagc 1380

agtcggttgg agcgagcatc ccccaaagtt cacaatgtgg ccgaggactt tgattgcaca 1440

ttgttgtttt tttaatagtc attccaaata tgagatgcgt tgttacagga agtcccttgc 1500

catcctaaaa gccaccccac ttctctctaa ggagaatggc ccagtcctct cccaagtcca 1560

cacaggggag gtgatagcat tgctttcgtg taaattatgt aatgcaaaat ttttttaatc 1620

ttcgccttaa tactttttta ttttgtttta ttttgaatga tgagccttcg tgccccccct 1680

tccccctttt ttgtccccca acttgagatg tatgaaggct tttggtctcc ctgggagtgg 1740

gtggaggcag ccagggctta cctgtacact gacttgagac cagttgaata aaagtgcaca 1800

ccttaaaaat gaaaaaaaaa aaaaaaaaaa aaaaaaaaaa aaaaaaaaaa aa 1852

<210> 5
<211> 1045
<212> DNA
<213> Homo sapiens

<400> 5
actatccggc gccgagccgg aggggggaaa cggcgcccgc cgcccgcccg gagcccgcga 60

gcaaccccag tcccccccac ccgcgcgtgg cggcgccggc tccctagcca ccgcggcccc 120

accctcttcc ggcctcagct gtccgggctg ctttcgcctc cgcctgtgga tgctgcgcct 180

ctccgaacgc aacatgaagg tgctccttgc cgccgccctc atcgcggggt ccgtcttctt 240

cctgctgctg ccgggacctt ctgcggccga tgagaagaag aaggggccca aagtcaccgt 300

caaggtgtat tttgacctac gaattggaga tgaagatgta ggccgggtga tctttggtct 360

cttcggaaag actgttccaa aaacagtgga taattttgtg gccttagcta caggagagaa 420

aggatttggc tacaaaaaca gcaaattcca tcgtgtaatc aaggacttca tgatccaggg 480

cggagacttc accaggggag atggcacagg aggaaagagc atctacggtg agcgcttccc 540

cgatgagaac ttcaaactga agcactacgg gcctggctgg gtgagcatgg ccaacgcagg 600

caaagacacc aacggctccc agttcttcat cacgacagtc aagacagcct ggctagatgg 660

caagcatgtg gtgtttggca aagttctaga gggcatggag gtggtgcgga aggtggagag 720

caccaagaca gacagccggg ataaacccct gaaggatgtg atcatcgcag actgcggcaa 780

gatcgaggtg gagaagccct ttgccatcgc caaggagtag ggcacaggga catctttctt 840

tgagtgaccg tctgtgcagg ccctgtagtc cgccacaggg ctctgagctg cactggcccc 900

ggtgctggca tctggtggag cggacccact cccctcacat tccacaggcc catggactca 960

cttttgtaac aaactcctac caacactgac caataaaaaa aaatgtgggt tttttttttt 1020

ttaatataaa aaaaaaaaaa aaaaa 1045

## Description

The present invention relates to a combination of markers for use in predicting the mortality risk in a patient suffering from polytrauma. The present invention also relates to a kit for predicting the mortality risk in such patient, and to a method of predicting the mortality risk in such patient. Furthermore, the present invention relates to a nucleic acid composition, to a matrix on which such nucleic acid composition has been immobilized. Furthermore, the present invention relates to uses of the aforementioned kit, of the nucleic acid composition and of the matrix.

Patients after multiple, severe trauma suffer from posttraumatic immune system destabilization and subsequent multiple organ dysfunction. This closely influences the patients' further clinical development. The Federal Bureau of Statistics for Germany indicates 88382 severely injured casualties in the year 2002 in Germany. The average age of these is approximately 40 years which means that these patients are in the midst of their working career. Multiply and severely traumatized patients who survived the initial posttraumatic resuscitation period suffer from substantial destabilization of their immune system. This phenomenon has been termed "systemic inflammatory response syndrome" (SIRS), which may lead to multiple organ dysfunction (and possibly failure) and thereby clearly impacts morbidity and mortality after multiple injury. Although these pathological immune reactions affect both components, namely the adaptive and innate immune system, the latter seems to play a superior role in the development of subsequent posttraumatic SIRS and posttraumatic multiple organ failure syndrome.

The admission into hospital and subsequent surgical management of polytraumatized patients involves a complex and prioritized approach. The central question in such management is which invasive method should be used in order to address each specific problem of the patient. On the one hand, a swift and thorough treatment of the individual injury needs to be secured, on the other hand, a significant deterioration of the general situation of the patient through further trauma, this time caused by surgical procedures, needs to be avoided. The determination of the therapeutic strategy and of the chances of success is based on a prediction of the extent of inflammation in the body. Clinical parameters could, so far, only be insufficiently used to make such predictions, since the initial intercellular mechanisms in human immune cells of polytraumatized patients are only poorly understood.

In the past, various clinical parameters have been used for making predictions of the clinical outcome of a patient. One example is interleukin-6 which has clinically been used for such purpose. However, all of these parameters, including interleukin-6 are not specific for polytraumatized patients and are subject to many other influences.

In previous genome-wide mRNA expression studies, the present inventors identified 763 genes which appear to be differentially expressed in polytraumatic patients. (Biberthaler et al., Shock, Vol. 24, No. 1, pp. 11-19, 2005; Bogner et al., Langenbecks Arch. Surg., 2007, 392: pp. 255-265). However, the teachings of these preliminary results remain very unspecific and do not point into a particular direction with respect to individual markers or specific combinations of markers that might be suitable for predicting the clinical outcome. Moreover, no teachings or suggestions are made with respect to markers that would be particularly suitable for predicting the outcome already at an early stage, e.g. a few hours, after onset of trauma. Therefore, there remains a need for a method that would allow to predict the clinical outcome of polytraumatized patients at an early stage.

Accordingly, it was an object of the present invention to provide for markers that allow to predict the clinical outcome of polytraumatized patients at a very early stage after onset of the polytrauma. It was also an object of the present invention to provide for markers which can be swiftly and easily determined in polytraumatized patients.

All these objects are solved by the use of a combination of markers, said markers being selected from the group comprising BCL2A, MMP-9 and ETS-2, for predicting the mortality risk in a patient suffering from polytrauma.

In one embodiment said combination is one of the following combinations:
a) BCL2A and MMP-9
b) BCL2A and ETS-2
c) MMP-9 and ETS-2
d) BCL2A, MMP-9 and ETS-2.

In one embodiment
- BCL2A is encoded by or is a nucleic acid, said nucleic acid being represented by SEQ ID NO: 1,
- MMP-9 is encoded by or is a nucleic acid, said nucleic acid being represented by SEQ ID NO: 2, and
- ETS-2 is encoded by or is a nucleic acid, said nucleic acid being represented by SEQ ID NO: 3.

In one embodiment said use comprises the quantitative determination of expression levels of said combination of markers in a body fluid of said patient.

In one embodiment said body fluid is blood, urine, lymphatic fluid, saliva or spinal fluid.

In one embodiment said use comprises the quantitative determination of expression levels of said combination of markers in leukocytes, preferably granulocytes, more preferably neutrophil granulocytes of said patient.

In one embodiment elevated expression levels of said combination of markers are indicative of an increased mortality risk.

In one embodiment said quantitative determination of expression levels is performed on samples of body fluid of said patient, said samples having been withdrawn from said patient at 60 - 120 minutes after onset of said polytrauma, and/or thereafter at 2 - 3 h and/or 3 - 4 h and/or 4 - 5 h and/or 5- 6 h and/or 6 - 7 h and/or 7 - 8 h and/or 8 - 9 h and/or 9 - 10 h and/or 10 - 11 h and/or 11 - 12 h after onset of said polytrauma. Additionally, said quantitative determination of expression levels may also be performed on samples of body fluid of said patient, said samples having been withdrawn at the onset of said polytrauma, or shortly thereafter. This may also sometimes herein be referred to as "0h".

Additionally, in one embodiment, samples are withdrawn from said patient at 5-7 h and/or 11-13 h and/or 23-25 h and/or 47-49 h and/or 71-73 h after onset of said polytrauma. In a preferred embodiment, samples are withdrawn from said patient at 6 h and/or 12 h and/or 24 h and/or 48 h and/or 72 h after onset of said polytrauma.

In one embodiment said determination of expression levels of said combination of markers occurs by detecting mRNA transcripts and/or proteins of said markers.

The objects of the present invention are also solved by a kit for predicting the mortality risk in a patient suffering from polytrauma, said kit comprising:
means to perform a quantitative determination of expression levels of a combination of markers, said combination of markers being as defined above, said quantitative determination of expression levels being as defined above.

In one embodiment said means are a set of primers for each marker to be determined, respectively, or a composition of antibodies, each antibody being specific for a marker to be determined.

The primers are used for performing nucleic acid complification reactions, such as PCR, and can be designed without difficulties by someone skilled in the art, based on the sequence(s) of the respective marker. More specifically, the design of the respective primers may, as an example, be performed with respect to the following amplicon regions (nucleotide positions with reference to sequence in sequence listing of this application):

| | | |
|---|---|---|
| BCL2-A | nucleotide positions | 509-746, |
| MMP-9 | nucleotide positions | 1984-2159, |
| ETS-2 | nucleotide positions | 1157-1454, |
| actin beta | nucleotide positions | 215-543, |
| cyclophilin B | nucleotide positions | 286-538. |

Other amplicon regions are also possible and can be determined by someone skilled in the art, based on the full-length sequence of the respective marker, as shown in the enclosed sequence listing. Such primers may also be obtained commercially and have, e.g., in the present case, been obtained from SEARCH-LC GmbH, Heidelberg. These commercially obtained primers were based on the afore-mentioned amplicon regions.

In one embodiment said kit further comprises reagents to perform nucleic acid amplification, and means to detect and quantitate amplified reaction products.

In one embodiment said kit further comprises reagents to perform an ELISA-assay, to detect and quantitatively determine expression levels of said combination of markers.

The objects of the present invention are also solved by a nucleic acid composition consisting of
a) two or three different nucleic acids, said nucleic acids coding for BCL2A, MMP-9, and ETS-2, respectively, and/or
b) the complementary counterparts thereof, and/or
c) partial sequences of a) and/or b), said partial sequences consisting of 15 contiguous nucleotides or more.

In one embodiment said nucleic acids coding for BCL2A, MMP-9, and ETS-2, respectively, are represented by SEQ ID NO: 1-3, respectively.

The afore-mentioned nucleic acid composition may be used in different applications. For example, if the nucleic acid composition consists of partial sequences of the nucleic acids coding for BCL2A, MMP-9 and ETS-2 (a), and partial sequences of the complementary counterparts of said coding nucleic acids (b), these partial sequences may be acting as primers in a nucleic acid amplification reaction. In another embodiment, the nucleic acid composition may be simply used as probes, either in solution or attached to some matrix. In this instance, such probes may comprise the coding sequences in accordance with a) and/or complementary counterparts thereof, and/or partial sequences of a) and/or b), which partial sequences may act as specific probes. In order for these to act as specific probes or primers, they must be of a sufficient length in order to achieve specific hybridization. In many instances, this can be achieved by sequence stretches of 12 nucleotides or more, such as 13, or 14, or 15, or 16, or 17, or 18, or 19, or 20, or 21, or 22, or 23, or 24, or 25, or 26 nucleotides.

The objects of the present invention are also solved by a solid matrix having a surface on which a nucleic acid composition as defined above has been immobilized.

In one embodiment said matrix is a substrate having a planar surface on which said nucleic acid composition has been immobilized.

It should be noted that in the afore-mentioned matrix-embodiments, i.e. those embodiments, wherein the nucleic acid composition as defined above has been immobilized on the surface of a matrix, there may be additional nucleic acids immobilized on said matrix, with the proviso that said matrix is not a genome-wide array of nucleic acid probes. In one embodiment of said matrix, said additional nucleic acids which may be immobilized on the surface of said matrix are not a complete collection of nucleic acid probes representing the entire genome of an organism, such as a human. In one embodiment of said matrix, said nucleic acids which may be immobilized on a surface of said matrix are selected from a group of genes consisting of housekeeping genes and partial sequences thereof. Such housekeeping genes are constitutively expressed genes, and are exemplified by beta actin and cyclophilin B. Other examples can be found in Trends in Genetics 19, 362-365 (2003). Whenever in such matrix embodiments housekeeping genes or partial sequences thereof (or the respective complementary counterparts thereof) are immobilized on a surface of the matrix in addition to the afore-mentioned nucleic acid composition, the nucleic acid matrix still does not have probes for the entire genome of an organism immobilized on its surface.

In one embodiment said matrix is a set of beads suitable for use in a high-throughput assay, said beads having surfaces on which said nucleic acid composition as defined above has been immobilized.

Similar remarks apply to this matrix-embodiment of a set of beads, with respect to the possibility of having additionally housekeeping genes or probes therefore immobilized on the surfaces of said beads. Again, altogether, the probes immobilized on the surfaces of the beads do not represent probes for the entire genome of an organism.

The objects of the present invention are also solved by the use of the kit according to the present invention, or of the composition according to the present invention, or of the matrix according to the present invention, for predicting the mortality risk in a patient suffering from polytrauma, wherein said use comprises the quantitative determination of a combination of markers, as defined above, or of said nucleic acid composition as defined above, wherein elevated expression levels of said combination of markers or of said nucleic acid composition are indicative of an increased mortality risk.

The objects of the present invention are also solved by a method of predicting the mortality risk in a patient suffering from polytrauma, said method comprising:
quantitatively determining the expression levels of at least two markers selected from the group comprising BCL2A, MMP-9, ETS-2 in a body fluid of said patient,
wherein elevated expression levels of said at least two markers are indicative of an increased mortality risk.

The term "polytrauma", as used herein, is meant to refer to a state of a patient who has suffered from multiple injuries, with at least one injury or a combination of injuries being life threatening.

The terms "BCL2A", "MMP-9" and "ETS-2", as used herein, refer to the respective genes on the nucleic acid level (DNA or RNA) and on the protein level. Therefore, if e.g. BCL2A is used for predicting the mortality risk in a patient, this means that such use may occur on the nucleic acid level, for example by determining the level of mRNA transcripts, or it may occur on the protein level, by determining the level of BCL2A-protein. The same applies to MMP-9 and ETS-2.

In one embodiment, said elevated expression levels indicative of an increased mortality risk are BCL2A-expression levels which are 1.3-fold to 3-fold of the BCL2A-expression levels of a polytraumatized patient who survives said polytrauma; and/or said elevated expression levels are MMP-9-expression levels which are 1.3-fold to 4-fold of the MMP-9-expression levels of a polytraumatized patient who survives said polytrauma; and/or said elevated expression levels are ETS-2-expression levels which are 1.1-fold to 3-fold of the ETS-2-expression levels of a polytraumatized patient who survives said polytrauma. In one embodiment, said elevated levels are determined by PCR.

In another embodiment, said elevated expression levels indicative of an increased mortality risk are BCL2-A expression levels which, at 5-7 h after onset of said polytrauma in said patient, are at least 2.5-fold the BCL2-A expression levels at the onset of said polytrauma in said patient; and/or said elevated expression levels indicative of an increased mortality risk are MMP-9 expression levels which, at 5-7 h after onset of said polytrauma in said patient, are at least 2.5-fold the MMP-9 expression levels at the onset of said polytrauma in said patient; and/or said elevated expression levels indicative of an increased mortality risk are ETS-2 expression levels which, at 5-7 h after onset of said polytrauma in said patient, are at least 5-fold the ETS-2 expression levels at the onset of said polytrauma in said patient.
Preferably said expression levels are determined by PCR. "at the onset of said polytrauma", as used herein, is meant to refer to the time point "0 h".

A "quantitative determination of expression levels", as used herein, is performed by measuring the levels of transcripts of the respective markers and/or by measuring the levels of expressed protein of the respective marker. Such quantitative determination is performed on samples which have been withdrawn from the patients already at an early stage. Prior to such quantitative determination, the respective sample may have to be processed such as further purified, concentrated, etc. In a preferred embodiment, such quantitative determination is performed on a body fluid of said patient, preferably on blood. In a more specific embodiment, the quantitative determination is performed on individual cell populations within said body-fluid, such as leukocytes, preferably granulocytes, preferably neutrophils.

The present inventors have surprisingly found that BCL2A and MMP-9 and ETS-2 show a highly significant correlation of their expression with the later clinical outcome of the respective patient. In other words, if these markers are expressed at an elevated level within a few hours after onset of trauma (typically 6 hours), this elevated expression correlates with a higher mortality of the respective patient than in a polytraumatized reference patient whose expression levels are not elevated at the same time. It is of vital importance that, for these markers, the elevated expression can already be determined very early, e.g. 6 hours after onset of trauma. Accordingly, these markers or their combination are particularly suitable for making a very early prognosis, namely at a point in time which is of extreme importance for planning the further therapy of the patient. The expression data are statistically correlated with the mortality of the patient using Receiver Operating Curves (ROC). For the entire data set, sensitivity and specificity of the individual marker are calculated with respect to its diagnostic potential for the clinical event. The 1-specificity value is plotted on the x-axis and the sensitivity value on the y-axis, and the individual data points are connected, thus forming a curve. The integral of this curve corresponds to the area under the curve (AUC), which is considered to be a significant discriminating tool as of values of 0.7 (or greater). For BCL2A, the ROC-analysis yields an error probability of p<0.0001, already at a time point of 6 h after onset of the polytrauma (AUC: 78 %, 95 % confidence interval: 0.63 - 0.93). With respect to MMP-9 at the time point of 6 h after onset of polytrauma, the error probability is at p<0.001 (AUC: 75 %, 95 %-confidence interval: 0.58 - 0.93). With respect to ETS-2 at the time point of 6 h after onset of polytrauma, the error probability is p<0.016 (AUC: 71 %, 95 %-confidence interval: 0.52 - 0.90) BCL2A, MMP-9 and ETS-2 have, so far, not been used as parameters for prognosis of clinical outcome in polytraumatized patients.

In accordance with the present invention, for the markers BCL2A, MMP-9 and ETS-2, the expression levels of these markers are quantitatively determined. Such determination is typically performed on a sample of body fluid of the patient, which has been withdrawn early after onset of polytrauma, typically not more than 90 minutes and in repeated intervals thereafter, e.g. 2h, 3h, 4h, 5h, 6h, 7h, 8h, ... and so forth ..., 72 h, ....96 h and so forth. Additionally, a determination may also be performed, where possible, on a sample of body fluid of the patient which has been withdrawn as early as possible after onset of polytrauma in said patient, e.g. a few minutes after onset, such as 5 minutes or 10 minutes. This is also meant to be included by the term "a sample withdrawn at 0 h".

A person skilled in the art knows how to quantitatively determine expression levels. For example, this may be done by performing quantitative real-time PCR using primer sets which are specific for the individual markers. Alternatively, ELISAs or western blots may be performed for such quantitative determinations.

The samples on which this quantitative determination is performed are typically body fluids from a patient, more typically blood. In one embodiment, such blood is subjected to further purification steps before a quantitative determination is performed. Such purification steps typically are directed at getting rid of impurities ore cellular components which are of no relevance for the quantitative determination. They may also be directed at the isolation of specific cell populations which are particularly suitable for a quantitative determination of the respective markers, such as neutrophil granulocytes. Whilst in the specific description, hereafter, an example is given wherein neutrophils are used for such quantitative determination, the present invention is, by no means, limited to quantitative determination in such neutrophils. For example, a quantitative determination may also be performed in blood serum, whole blood, leukocytes, granulocytes, monocytes and liquor.

Typically, the quantitative determination of the expression levels of the respective markers includes a detection step. A person skilled in the art knows how to perform such detection step, and preferred means involve the use of fluorescent dyes which may be used to detect amplified expression products, if for example real-time PCR is used, or which may be used to detect antibody-marker-complexes, if ELISAs or western blots are used. A typical example of such a fluorescent dye is SYBR green, which can be used in real-time PCR protocols.

Another possibility for quantitatively determining expression levels is the use of an array in which probes specific for the respective markers are immobilized on a matrix, such as a substrate having a surface, or beads on the surface of which the respective probes are immobilized. It should be noted that, in accordance with the present invention, an array of such marker-specific probes does not comprise probes for the entire genome. Consequently, a typical array in accordance with the present invention comprises a nucleic acid composition consisting of two or three different nucleic acids only, which are specific for the individual markers. The nucleic acid composition does not comprise further different nucleic acids, with the exception of one to ten reference nucleic acids as a control. Such controls may e.g. be housekeeping genes. The term "specific for an individual marker", when used herein in connection with a nucleic acid or a nucleic acid probe, is meant to refer to a nucleic acid which has a nucleic acid sequence that is specific for such marker, or which has a nucleic acid sequence that hybridizes under stringent conditions to the nucleic acid sequence of said marker. The term "two or three different nucleic acids" is meant to refer to two or three nucleic acids having sequences which differ from each other.

The markers and combination of markers in accordance with the present invention have the unprecedented advantage of being highly and significantly predictive for the mortality and clinical outcome of a polytraumatized patient, especially at an early time after onset of said trauma, for example already at 6 h after onset of said trauma. This is unprecedented and was nowhere suggested or described in the prior art before. It opens up a new perspective for treatment and treatment management of polytraumatized persons at a very early stage of their multiple injuries and thus greatly improves prospects of treatment for such patients.

Moreover, reference is made to the enclosed sequence listing, wherein
SEQ ID NO: represents the sequence for BLC2A,
SEQ ID NO:2 represents the sequence for MMP-9,
SEQ ID NO:3 represents the sequence for ETS-2.
SEQ ID NO: 4 represents the sequence for actin beta, and
SEQ ID NO: 5 represents the sequence for cyclophilin B.

Moreover, reference is made to the following figures, wherein

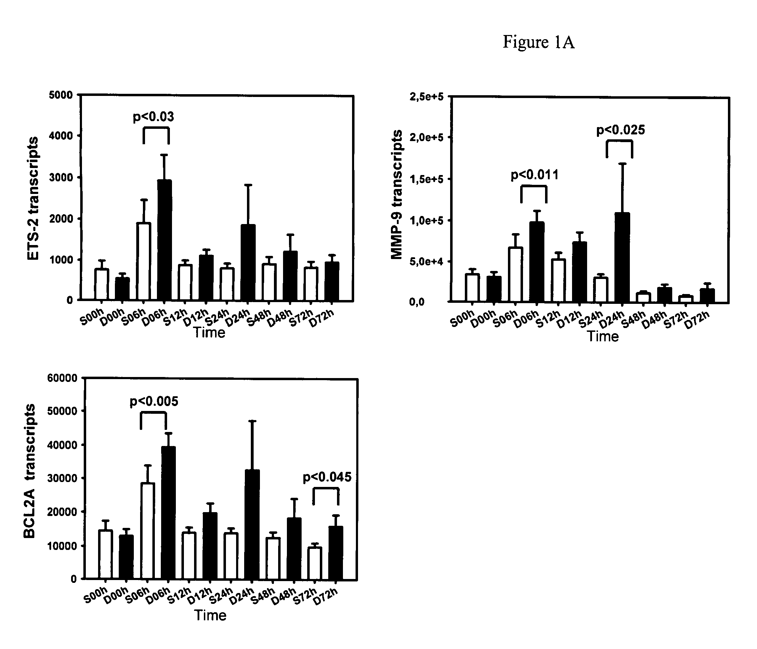

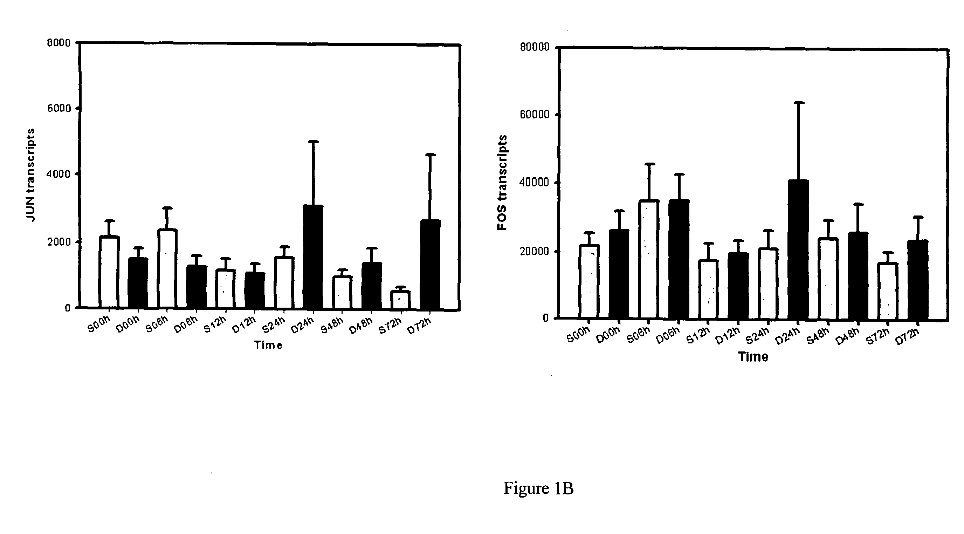

### Figure 1: shows outcome related differential gene expression

These plots visualize the differential gene expression of (a) ETS-2, BCL2A, and MMP-9, and b) JUN and FOS, depending on the patients' clinical outcome. JUN and FOS exemplify markers not showing significant expression differences. The white bar charts depict the mean (±SEM) gene transcripts in patients with favourable outcome (n=28) as compared to expression levels of the respective gene in patients who deceased (n=12). Significant differences are marked by the respecting p-values calculated by Mann-Whitney-Rank Sum Tests.

### Figure 2: shows outcome classifying ROCs:

This graph shows the potential of outcome classification using a) BCL2A, b) ETS-2, c) MMP-9, d) JUN and e) FOS transcript measurement in the posttraumatic period (0-72h) assessed by receiver operating curves (ROC).

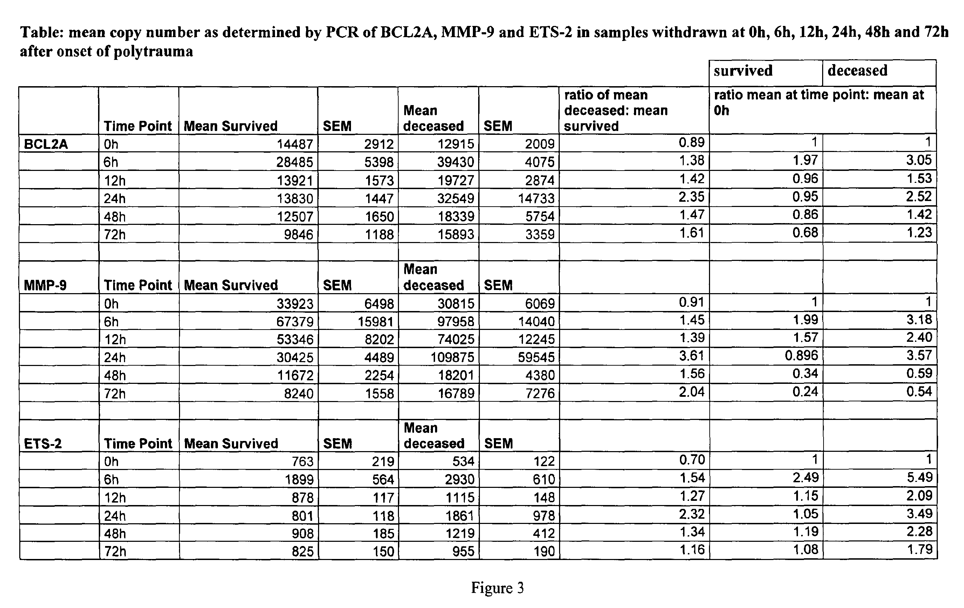

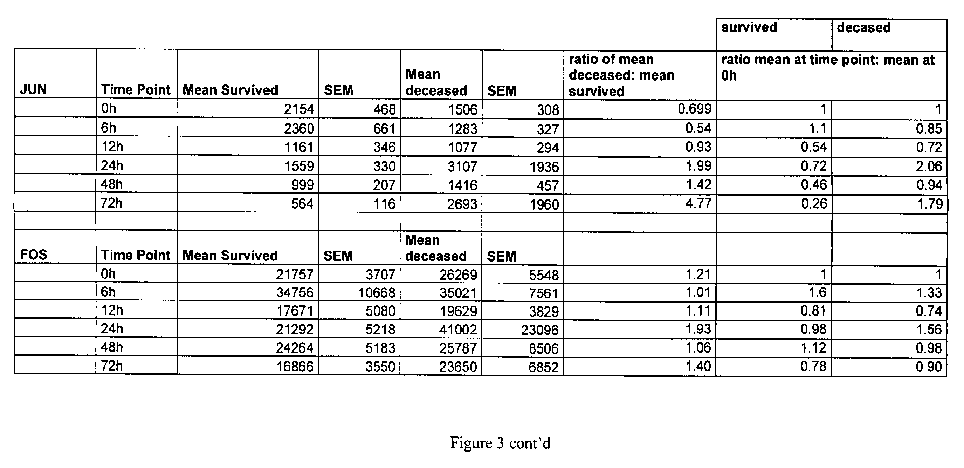

### Figure 3: shows a table outlining the mean copy numbers as determined by PCR of BCL2A, MMP-9, ETS-2, JUN and FOS:

The tables shows the mean copy numbers of BCL2-A, MMP-9 and ETS-2 in samples of blood withdrawn at 0 h, 6 h, 12 h, 24 h, 48 h and 72 h after onset of polytrauma; the values are classified according to clinical outcome (deceased = patients who died; survived = patients who survived). Copy numbers were normalized using the ratios of housekeeping genes actin and cyclophilin B (CPB). Also shown is the ratio of mean copy numbers in deceased patients to mean copy numbers in surviving patients at the individual time points. Also shown is the ratio of the mean at time point x to the mean at time point 0h.

Moreover, reference is made to the following examples which are given to illustrate, not to limit the present invention:

### Examples

### Example 1

### Patients and Methods:

This investigation was conducted in our level 1 trauma center strictly according to the Good Clinical Practice Guidelines. Ethical innocuousness was certified by the local institutional review board (reference number: 012/00). Patients of full age suffering from blunt multiple injuries [Injury Severity Score (ISS) of >16 points (9)] who were admitted to our emergency department within 90 minutes after trauma were included into the study. Consent form of study participation was obtained from the patients or a legal representative. Patients who departed within the first 24h after trauma were excluded from the study. Patients' treatment was performed according to standard of care and was not affected by study participation. The patient collective was retrospectively classified into opposed clinical groups according to their injury severity (ISS), injury pattern concerning the presence of traumatic brain injury (positive initial CCT scan), presence of massive red blood cell substitution unit and finally according to their ultimate clinical outcome (90-day survival). Application of more than 10 RBC units/24h has been defined as the presence of massive RBC transfusion (10).

### Blood sampling and neutrophil isolation

Blood samples were drawn on admission of the patient - but purposefully not later than 90 minutes after trauma - and below at 6h, 12h, 24h, 48h and 72h. Sampling time points are strictly standardized to the traumatic event. After three rounds of red blood cell lysis, the samples were centrifuged at 450 x g. The resulting cell pellets were resuspended in PBS/EDTA buffer. 200µl anti-CD15 antibody coated micromagnetic beads (Miltenyi Biotech, Auburn, CA, USA) were added to the 1 x 10⁸ cells diluted in 800 µl PBS/EDTA buffer for 15 minutes at 4° C. After pelleting and dilution, the cells were added to a pre-equalized high gradient magnet separation column using the magnetic cell separator Mini-MACS™ system (Miltenyi Biotech). The cell suspension was lysed in 2 ml of RLT buffer (Qiagen, Hilden, Germany) containing 0.1 % v/v β-mercaptoethanol and stored at -80° C. Total RNA preparation from lysed neutrophils was done by the RNeasy Midi Kit™ according to the supplier's recommendations (Qiagen). DNA was removed by on-column DNA-se digestion using the MiniElute™ Clean Up Kit (Qiagen).

### cDNA Synthesis and Quantitative Real-Time PCR

mRNA preparation was performed using the RNeasy Midi Kit (Qiagen, Hilden). cDNA was prepared with a first-strand cDNA synthesis kit; and QRT-PCR was performed with the Light Cycler Fast Start DNA SYBR Green kit, all according to the manufacturer's instructions (Roche, Mannheim). All primers, including such of the housekeeping genes were derived from Search-LC (Heidelberg, Germany). Primers were based on the following amplicon regions of the respective full-length sequence, as shown in the sequence listing:
BCL2-A: nucleotide positions 509-746
MMP-9: nucleotide positions 1984-2159
ETS-2: nucleotide positions 1157-1454.
actin beta: nucleotide positions 215-543
cyclophilin B: nucleotide positions 286-538.
Detected transcript amounts were normalized using the ratios of housekeeping genes actin and cyclophilin B (CPB).

### Statistical Analyses

Analyses of expression comparison between the clinical groups have been performed by Mann-Whitney-Rank Sum Test for non-normal variables and t-tests for normal variables (SigmaStat®, SPSS. Inc.). Visualization of the data was performed by Sigma Plot 8.0 (SPSS. Inc.). Data are given as meant SEM. The outcome and massive transfusion classifying values of the different genes were assessed by Receiver Operating Curves (ROC; Analyse IT, Windows). ROC curve results are given as p-values, area under the curve (AUC) and 95% confidence interval.

### Example 2

### Results:

### Patients and Methods:

40 patients (28 males and 12 females) fulfilled the entry criteria and were included into the study. Patients' age ranged from 18 to 71 years. Injury Severity Score in the entire study collective counted 36±14 points, mean New Injury Severity Score (NISS) 41±13 points (mean±SD). The injury pattern of 15 patients included severe traumatic brain injury (intracranial lesion in the initial CCT scan). 28 of the collective survived the trauma, whereas 12 had an unfavourable outcome. Thereof, 6 patients deceased because of severe traumatic brain injury and 6 patients died of multiple organ failure in the further posttraumatic clinical course. Mean ISS (±SD) in the patients who survived the traumatic event was 36±13 points (NISS 39±12), in those who deceased 37±17 points (NISS 46±14). During the observation period, all patients developed clinical signs of multiple organ failure (MOF score ≥ 4 points). 21 patients required massive RBC substitution and were resuscitated with 25,6±10 RBC units in contrast to the 19 patients who received less, namely 5,0±3 RBC units (mean±SD). 11 patients in the massive transfusion group deceased.

### Differential Gene Expression depending on clinical outcome

Patients who did not survive the traumatic event (90-day survival) exhibit significant higher BCL2A levels at all blood sampling time points. Significant differences can be observed already at 6h after trauma in comparison to patients with a favourable outcome p<0.005, ROC curve (data given as p, AUC and 95% confidence interval): p<0.0001; 0,78; 0,63-0,93. Also at 72h BCL2A expression changes are significantly higher in deceased patients p<0.045. Neutrophil MMP-9 expression exhibits significant differences in patients who decease as compared to those who survive at 6h: p< 0.011, ROC curve p<0.001; 0,75; 0,58-0,93 and at 24h post trauma: p<0.025, ROC curve p<0.011; 0,72; 0,53-0,92.

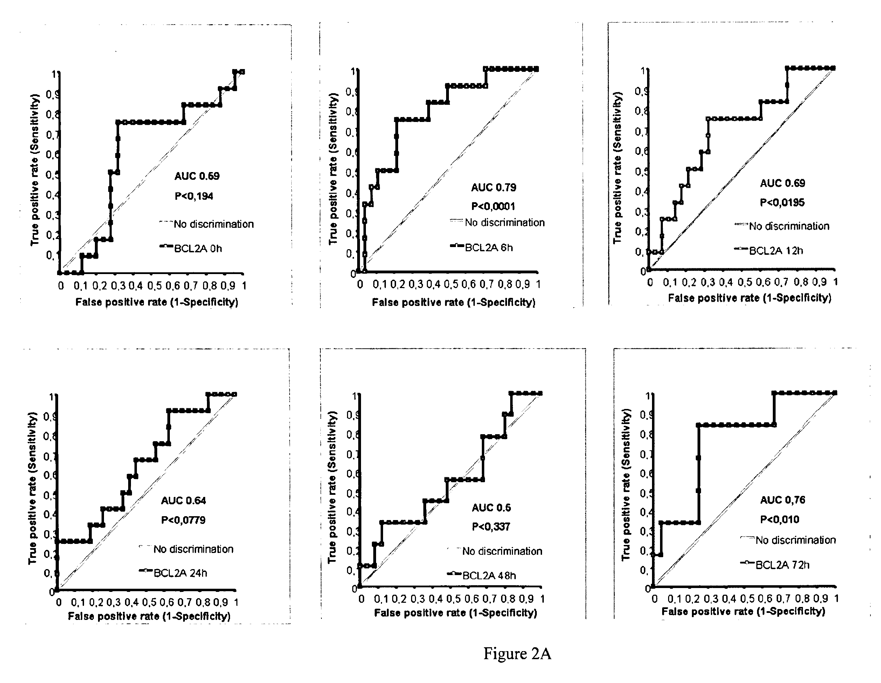

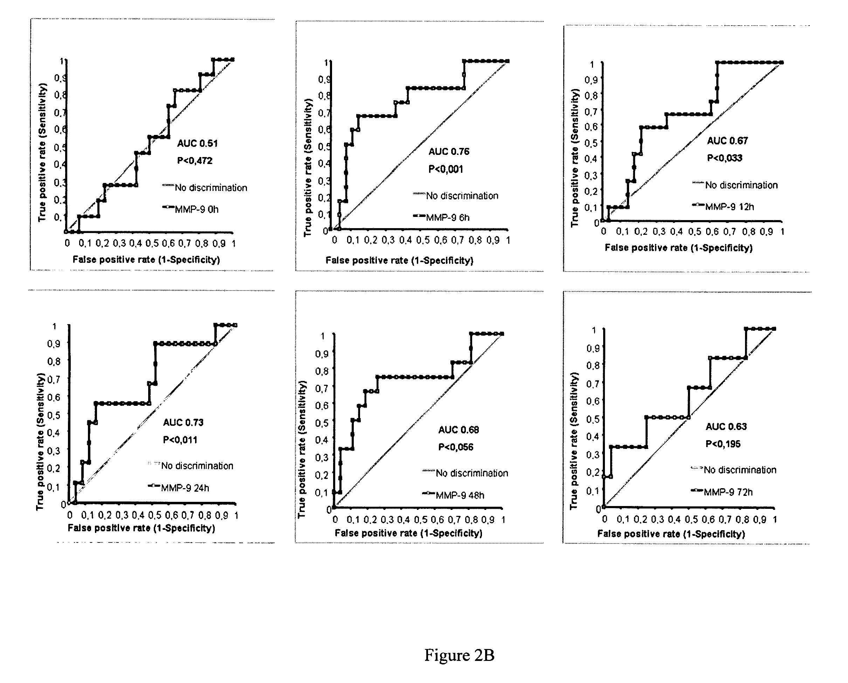

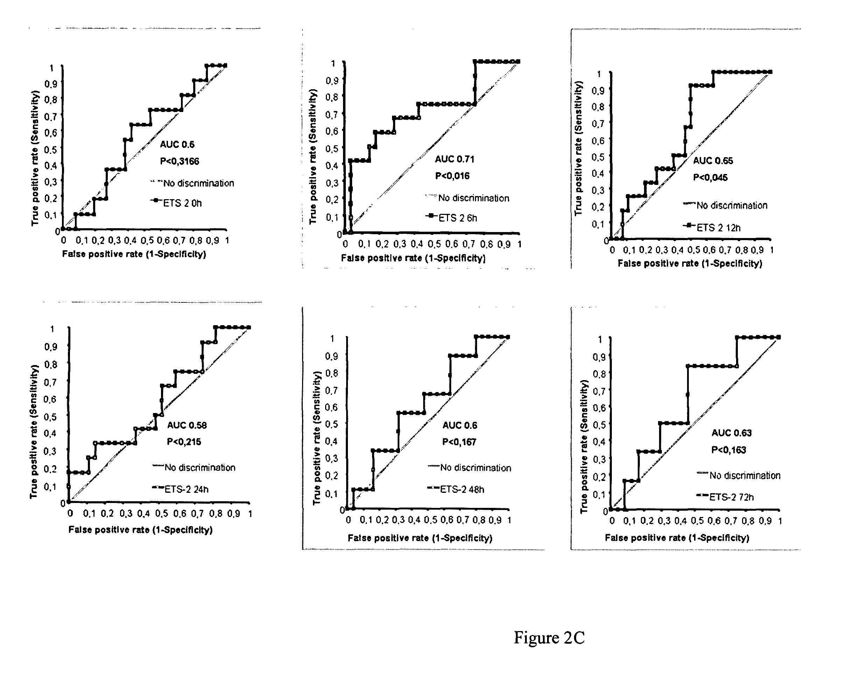

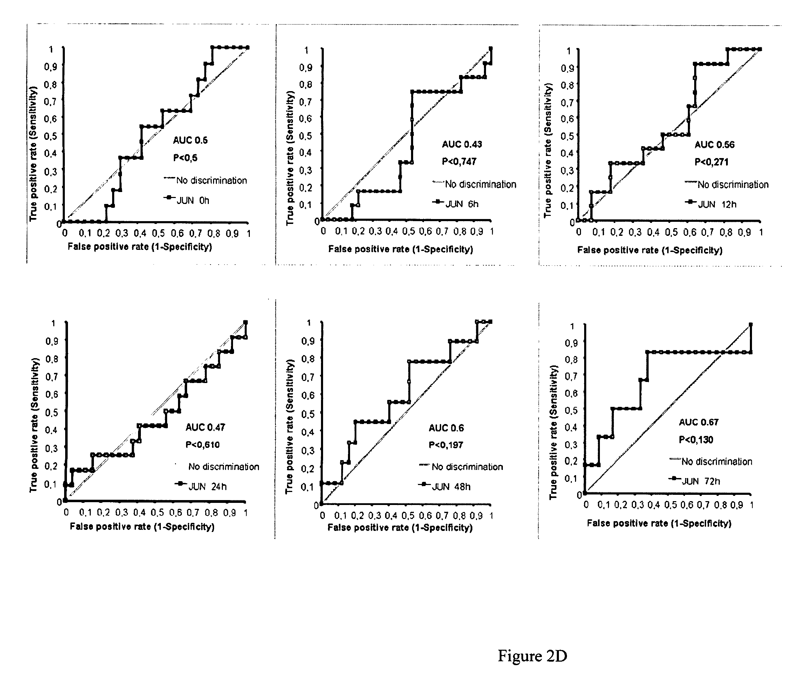

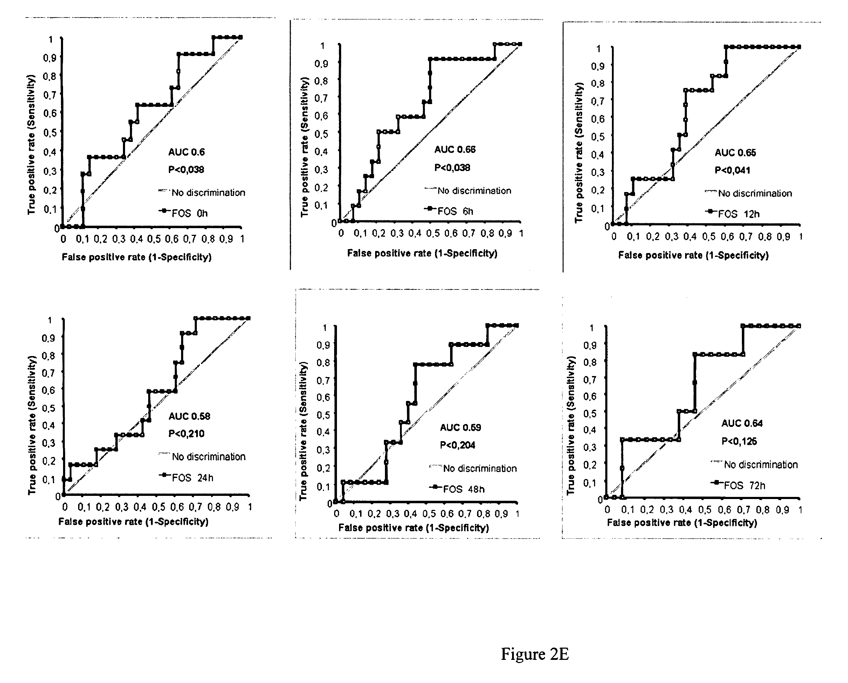

ETS-2 expression also shows higher expression at 6h after trauma in patients with unfavourable outcome p<0.03, ROC: p<0.01, 0,71; 0,51-0,90. The outcome related plots are visualized in **figure 1A****,** the respecting ROC curves in **figure 2****.**

Figure 1B shows two examples of markers (JUN and FOS) which do not show significant differences in expression depending on clinical outcome, thus illustrating the suitability and superiority of BCL2A, MMP-9 and ETS-2 for predicting clinical outcome, in comparison to markers like JUN and FOS.

Altogether, the present invention has identified new combinations of markers consisting of at least two from BCL2 A, MMP-9, and ETS-2, which are differentially upregulated and over-expressed very early after the onset of polytrauma in such patients with a fatal clinical outcome (non-survival within 90 days after polytrauma). This means that use of these markers and combinations thereof enables new approaches for treatment of such patients who are at a higher mortality risk, in choosing less invasive treatments, where possible, to avoid additional risks posed by surgical measures.

Treatment of such patients has so far mainly relied upon the experience of the treating physician. Decisions on specific courses of treatments can now be taken based on the present invention at a very early stage after the patients have been admitted.

### Literature:

(1) Mannick JA, Rodrick ML, Lederer JA. The immunologic response to injury. J Am Coll Surg 2001; 193(3):237-244.
(2) Baue AE, Durham R, Faist E. Systemic inflammatory response syndrome (SIRS), multiple organ dysfunction syndrome (MODS), multiple organ failure (MOF): are we winning the battle? Shock 1998; 10(2):79-89.
(3) Angele MK, Faist E. Clinical review: immunodepression in the surgical patient and increased susceptibility to infection. Crit Care 2002; 6(4):298-305.
(4) Cioffi WG, Burleson DG, Pruitt BAJ. Leukocyte responses to injury. Arch Surg 1993; 128(11):1260-1267.
(5) Oberholzer A, Oberholzer C, Moldawer LL. Sepsis syndromes: understanding the role of innate and acquired immunity. Shock 2001; 16(2):83-96.
(6) Biberthaler P, Bogner V, Baker HV, Lopez MC, Neth P, Kanz KG et al. Genome-wide monocytic mRNA expression in polytrauma patients for identification of clinical outcome. Shock 2005; 24(1):11-19.
(7) Matsuda N, Hattori Y. - Systemic inflammatory response syndrome (SIRS): molecular pathophysiology and gene therapy.(3):-98.
(8) Xing L, Remick DG. - Relative cytokine and cytokine inhibitor production by mononuclear cells and neutrophils.(1):-6.
(9) Committe on Injury Scaling (1990). Abbreviated Injury Scale 1990 Revision. Des Plaines, Illinois: Association of the Advancement of Automovement Medicine, 2002.
(10) Como JJ, Dutton RP, Scalea TM, Edelman BB, Hess JR. Blood transfusion rates in the care of acute trauma. Transfusion 2004; 44(6):809-813.
(11) Foletta VC, Segal DH, Cohen DR. - Transcriptional regulation in the immune system: all roads lead to AP-1.(2):-52.
(12) Yao YM, Redl H, Bahrami S, Schlag G. - The inflammatory basis of trauma/shock-associated multiple organ failure.(5):-10.
(13) Pillay J, Hietbrink F, Koenderman L, Leenen LP. - The systemic inflammatory response induced by trauma is reflected by multiple phenotypes of blood neutrophils.(12):-72.
(14) Kreth S, Ledderose C, Kaufmann I, Groeger G, Thiel M. - Differential expression of 5'-UTR splice variants of the adenosine A2A receptor gene in human granulocytes: identification, characterization, and functional impact on activation.(9):-86.
(15) Dunne JR, Malone DL, Tracy JK, Napolitano LM. Allogenic blood transfusion in the first 24 hours after trauma is associated with increased systemic inflammatory response syndrome (SIRS) and death. Surg Infect (Larchmt ) 2004; 5(4):395-404.
(16) Malone DL, Dunne J, Tracy JK, Putnam AT, Scalea TM, Napolitano LM. Blood transfusion, independent of shock severity, is associated with worse outcome in trauma. J Trauma 2003; 54(5):898-905.
(17) Villunger A, Scott C, Bouillet P, Strasser A. - Essential role for the BH3-only protein Bim but redundant roles for Bax, Bcl-2, and Bcl-w in the control of granulocyte survival.(6):-400.
(18) Villunger A, O'Reilly LA, Holler N, Adams J, Strasser A. - Fas ligand, Bcl-2, granulocyte colony-stimulating factor, and p38 mitogen-activated protein kinase: Regulators of distinct cell death and survival pathways in granulocytes.(5):-58.
(19) Iwai K, Miyawaki T, Takizawa T, Konno A, Ohta K, Yachie A et al. - Differential expression of bcl-2 and susceptibility to anti-Fas-mediated cell death in peripheral blood lymphocytes, monocytes, and neutrophils.(4):-8.
(20) Maier M, Wutzler S, Bauer M, Trendafilov P, Henrich D, Marzi I. - ALTERED GENE EXPRESSION PATTERNS IN DENDRITIC CELLS AFTER SEVERE TRAUMA: IMPLICATIONS FOR SYSTEMIC INFLAMMATION AND ORGAN INJURY.:-Shock.
(21) Chow AK, Cena J, Schulz R. - Acute actions and novel targets of matrix metalloproteinases in the heart and vasculature.(2):-205.
(22) Oettgen P. - Regulation of vascular inflammation and remodeling by ETS factors.(11):-66.
(23) Wei G, Guo J, Doseff AI, Kusewitt DF, Man AK, Oshima RG et al. - Activated Ets2 is required for persistent inflammatory responses in the motheaten viable model.(2):-9.
(24) Hildebrand F, Pape HC, Krettek C. [The importance of cytokines in the posttraumatic inflammatory reaction]. Unfallchirurg 2005; 108(10):793-803.
(25) Liener UC, Bruckner UB, Knoferl MW, Steinbach G, Kinzl L, Gebhard F. - Chemokine activation within 24 hours after blunt accident trauma.(3):-72.
(26) Ni CN, Redmond HP. - Cell response to surgery.(11):-40.
(27) Gardner J, Ghorpade A. - Tissue inhibitor of metalloproteinase (TIMP)-1: the TIMPed balance of matrix metalloproteinases in the central nervous system.(6):-6.
(28) von Gertten C, Holmin S, Mathiesen T, Nordqvist AC. - Increases in matrix metalloproteinase-9 and tissue inhibitor of matrix metalloproteinase-1 mRNA after cerebral contusion and depolarisation.(6):-10.
(29) Wang X, Jung J, Asahi M, Chwang W, Russo L, Moskowitz MA et al. - Effects of matrix metalloproteinase-9 gene knock-out on morphological and motor outcomes after traumatic brain injury.(18):-42.

## Claims

1. Use of a combination of markers, said markers being selected from the group comprising BCL2A, MMP-9 and ETS-2, for predicting the mortality risk in a patient suffering from polytrauma.

2. Use according to claim 1, wherein said combination is one of the following combinations:
a) BCL2A and MMP-9
b) BCL2A and ETS-2
c) MMP-9 and ETS-2
d) BCL2A, MMP-9 and ETS-2

3. Use according to any of claims 1-2, wherein
- BCL2A is encoded by or is a nucleic acid, said nucleic acid being represented by SEQ ID NO: 1,
- MMP-9 is encoded by or is a nucleic acid, said nucleic acid being represented by SEQ ID NO: 2, and
- ETS-2 is encoded by or is a nucleic acid, said nucleic acid being represented by SEQ ID NO: 3.

4. Use according to any of claims 1-3, wherein said use comprises the quantitative determination of expression levels of said combination of markers in a body fluid of said patient.

5. Use according to claim 4, wherein said body fluid is blood, urine, lymphatic fluid, saliva or spinal fluid.

6. Use according to any of claims 4-5, wherein said use comprises the quantitative determination of expression levels of said combination of markers in leukocytes, preferably granulocytes, more preferably neutrophil granulocytes of said patient.

7. Use according to any of claims 4-6, wherein elevated expression levels of said combination of markers are indicative of an increased mortality risk.

8. Use according to any of claims 4-7, wherein said quantitative determination of expression levels is performed on samples of body fluid of said patient, said samples having been withdrawn from said patient at 60 - 120 minutes after onset of said polytrauma, and/or thereafter at 2h - 3h and/or 3h - 4h and/or 4h - 5h and/or 5h - 6h and/or 6h - 7h and/or 7h - 8h and/or 8h - 9h and/or 9h-10h and/or 10h - 11h and/or 11h - 12h after onset of said polytrauma..

9. Use according to any of claims 4-8, wherein said determination of expression levels of said combination of markers occurs by detecting mRNA transcripts and/or proteins of said markers.

10. A kit for predicting the mortality risk in a patient suffering from polytrauma, said kit comprising:
means to perform a quantitative determination of expression levels of a combination of markers, said combination of markers being as defined in any of claims 1-3, said quantitative determination of expression levels being as defined in any of claims 3-9.

11. Kit according to claim 10, wherein said means are a set of primers for each marker to be determined, respectively, or a composition of antibodies, each antibody being specific for a marker to be determined.

12. A nucleic acid composition consisting of
a) two or three different nucleic acids, said nucleic acids coding for BCL2A, MMP-9, and ETS-2, respectively, and/or
b) the complementary counterparts thereof, and/or
c) partial sequences of a) or b), said partial sequences consisting of 15 contiguous nucleotides or more, and/or
d) partial sequences of a) and b), said partial sequences consisting of 15 contiguous nucleotides or more.

13. Nucleic acid composition according to claim 12, wherein said nucleic acids coding for BCL2A, MMP-9, and ETS-2, respectively, are represented by SEQ ID NO: 1-3, respectively.

14. A solid matrix having a surface on which a nucleic acid composition as defined in any of claims 12-13 has been immobilized.
